# EUROPEAN PATENT APPLICATION

(11) **EP 4 541 329 A2**
(43) Date of publication of application: **23.04.2025**
(21) Application number: 23824105.3
(22) Date of filing: 19.05.2023
(51) Int. Cl.: A61F 5/455, A61F 5/44

(54) **MENSTRUAL CUP**

(30) Priority: 16.06.2022 KR 20220073552
(71) Applicant: Lee, Jisoo, Ansan-si, Gyeonggi-do 15399 (KR)
(72) Inventor: Lee, Jisoo, Ansan-si, Gyeonggi-do 15399 (KR)
(74) Representative: Cabinet Chaillot
(86) International application number: PCT/KR2023/006825
(87) International publication number: WO 2023/243884

(57) **Abstract**

The present invention relates to a menstrual cup, and more particularly, to a menstrual cup including a cup body formed in a shape of a bell with a hollow and an opening facing upward to relieves a sense of hesitation or reluctance, while not causing pain when inserted into a woman's vagina by allowing crumpling of an outermost point among edges formed when folded; wherein the cup body is formed with a flexible portion including the point located at the outermost side among the edges formed when folded and having a small thickness relative to other portions.

## Description

### [TECHNICAL FIELD]

The present invention relates to a menstrual cup, and more particularly, to a menstrual cup that may be folded in a form which is easily inserted into a woman's vagina and relieves a sense of hesitation or reluctance while not causing pain.

### [BACKGROUND]

In general, female hygiene/ menstrual products can be categorized as pad-type hygiene products that are used by being attached to the inside of underwear and insertable hygiene products that are used by being inserted into woman's vagina.

Although pad-type hygiene products have excellent absorbency and a small thickness, because they move together with the underwear when they are worn during an activity, there are limitations as to free activities such as exercise, and because they are disposable (one-time use), environmental problems and cost burden arise. Accordingly, the development and use of insertable hygiene products that are capable of solving the problems of pad-type hygiene products are increasing.

A menstrual cup is a representative example of an insertable hygiene product, and it includes a cup body that is bell-shaped with an opening facing upward and a handle that protrudes a certain length downward from a lower end of the cup body. Such a menstrual cup is inserted into the vagina by folding the opening and is fixed therein after the folded opening is unfolded by elasticity.

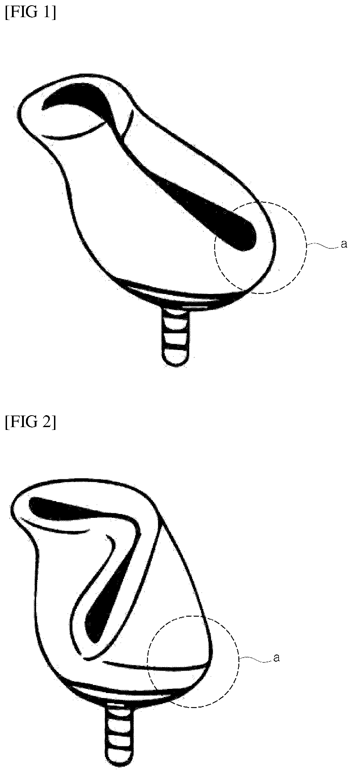

There are various methods of folding a menstrual cup, such as Punch down, 7-fold, and Diamond, etc. In a menstrual cup that are folded in these ways, there are problems: a point a located at an outermost side of edges formed when folded as shown in FIGS. 1 and 2 is relatively thick and pointed in shape and thereby causes pain or gives rise to a sense of hesitation or reluctance when inserted into the vagina.

### [Detailed Description of Invention]

### [Technical Problem]

To solve the above problems, an object of the present invention is directed to providing a menstrual cup that relieves a sense of hesitation or reluctance, while not causing pain when inserted into a woman's vagina by allowing crumpling of an outermost point among edges formed when folded.

### [Technical Solution]

To achieve the above objects, a menstrual cup of the present invention includes a cup body, which is formed in a shape of a bell with an empty interior and an opening facing upward, and the cup body is formed with a flexible portion including the point located at the outermost side among the edges formed when the cup body is folded and having a small thickness relative to other portions.

In addition, the cup body may include a rim portion at an upper end; a first receiving portion extending downward from the rim portion in a shape of a cylinder which is open from top to bottom; and a second receiving portion extending downward from the first receiving portion in a shape of a hemisphere which opens upward; wherein the flexible portion is formed to include a boundary point between the first receiving portion and the second receiving portion.

In addition, the flexible portion may be formed in a shape of a curved plate across the first receiving portion and the second receiving portion, and to have a length of less than 2/3 of a total length of the cup body, and in a plan view, to have a circumference of less than 1/2 of a total circumference of the cup body.

In addition, the flexible portion may be formed with an inner peripheral surface of the cup body recessed more than other parts or portions.

In addition, the flexible portion may be formed with an outer peripheral surface of the cup body recessed more than other parts or portions.

### [Advantageous Effects]

According to the present invention, when a cup body is folded to be inserted into a woman's vagina, a flexible portion that is relatively thin compared to other portions are crumpled, and the flexible portion, which is an outermost point of the edges formed as the cup body is folded does not protrude sharply or pointedly, pain is not caused when inserted into the vagina, and thus, discomfort and a sense of reluctance can be relieved.

### [Description of Drawings]

FIG. 1 is a perspective view showing a conventional menstrual cup folded using a punch-down method.
FIG. 2 is a perspective view showing a conventional menstrual cup folded using a 7-fold method.
FIG. 3 is a perspective view showing a menstrual cup of the present invention according to a first embodiment.
FIG. 4 is a cross-sectional view showing the menstrual cup of the present invention according to the first embodiment.
FIG. 5 is a plan view showing the menstrual cup of the present invention according to the first embodiment.
FIG. 6 is a perspective view showing the menstrual cup of the present invention according to a second embodiment.
FIG. 7 is a front view showing the menstrual cup of the present invention according to the second embodiment.
FIG. 8 is a side view showing the menstrual cup of the present invention according to the second embodiment.
FIG. 9 is a cross-sectional view showing the menstrual cup of the present invention according to the second embodiment.
FIG. 10 is a perspective view showing a state in which an elastic reinforcement rim is provided in a flexible portion applied to the menstrual cup of the present invention.
FIG. 11 is a perspective view showing the menstrual cup of the present invention that is folded using a punch-down method.
FIG. 12 is a perspective view showing the menstrual cup of the present invention that is folded using a 7-fold method.

### [Best Mode of the Invention]

In the present invention, a menstrual cup is proposed, wherein the menstrual cup includes a cup body, which is formed in a shape of a bell with a hollow and an opening directed upward, so that crumpling is possible at a point located at an outermost side among edges formed when folded to relieve a sense of reluctance while not causing pain when inserted into a woman's vagina, and wherein the cup body is formed with a flexible portion, including the point located at the outermost side among the edges formed when the cup body is folded and having a small thickness relative to other portions.

### [Modes of the Invention]

The scope of the present invention is not limited to embodiments described below, and various modifications and implementations may be made by those skilled in the art without departing from the technical spirit of the present invention.

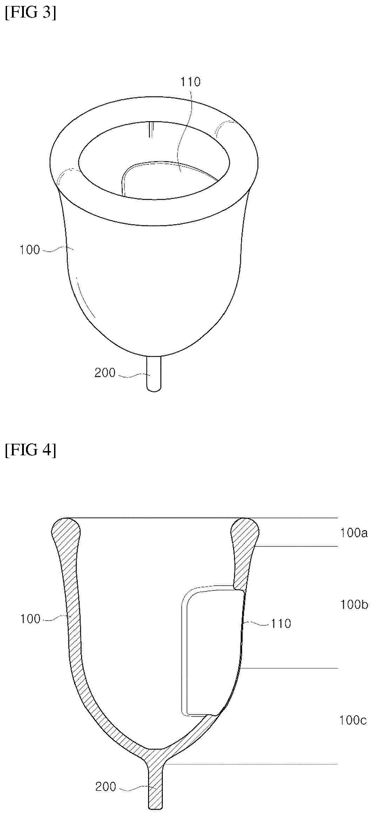

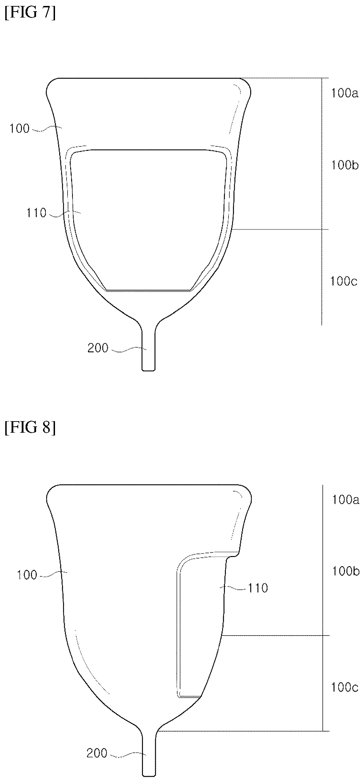

Hereafter, a menstrual cup of the present invention is described in detail with reference to FIGS. 3 to 12.

The menstrual cup of the present invention includes a cup body 100 which may accommodate or receive menstrual blood therein as shown in FIGS. 3 to 12 and may further include a handle 200 for a user to hold, which protrudes downward from a lower end of the cup body 100 to have a certain length.

The cup body 100 may be formed to have a bell shape with a hollow and an opening facing upward and formed in any of various sizes depending on a receiving capacity for menstrual blood. Also, the cup body 100 may be made of a soft and elastic material such as silicone, so that the user may fold the cup body 100 to the user's intention, and the folded part may be unfolded due to elasticity and restored to its original state. Accordingly, the cup body 100 may be folded in various ways, such as punch down, 7-fold, and diamond, etc., and inserted to position the opening in an innermost part of the woman's vagina.

The cup body 100 is formed with a flexible portion 110 having a smaller thickness than other portions, as shown in FIGS. 3 to 12. As an example, the thickness of the flexible portion 110 may be 1/20 to 1/3 of the thickness of other portions.

The cup body (100) may include a rim portion 100a at an upper end, a first receiving portion 100b extending downward from the rim portion 100a in a shape of a cylinder which is open from top to bottom, and a second receiving portion 100c extending downward from the first receiving portion 100b in a shape of a hemisphere which opens upward, as shown in FIG. 4, FIGS. 7 to 9. As a specific example, the rim portion 100a may be formed thicker than the first receiving portion 100b and the second receiving portion 100c so as to have a strong restoring force, and may be formed to have a thickness of 4 to 6 mm. Here, the first receiving portion 100b and the second receiving portion 100c may be formed to have a thickness of 2.5 to 3 mm, and the flexible portion 110 may be formed to have a thickness of less than 1 mm. Preferably, the flexible portion 110 may be formed to have a thickness of 0.3 to 0.5 mm.

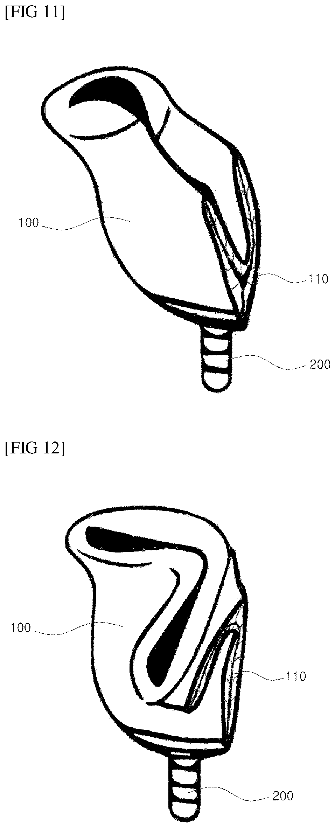

In this way, the flexible portion 110, which has a relatively smaller thickness than other parts or portions of the cup body 100, is formed at the cup body 100 so as to include the point located at the outermost side among the edges formed when folded to be inserted into the vagina. As an example, the flexible portion 110 may be formed to include a lower portion of the cup body 100, that is, the boundary point between the first receiving portion 100b and the second receiving portion 100c, so that when folded using the punch down or 7-fold method, etc., as shown in FIG. 11 and FIG. 12, the flexible portion 110, which is located at the outermost side among the edges folded, may be crumpled. As another example, the flexible portion 110 may be formed to include a portion of a middle portion of the cup body 100, that is, a portion of the first receiving portion 100b, so that when folded using the diamond method, etc., the flexible portion 110, which is located at the outermost side among the edges folded, may be crumpled.

Among the various ways to fold a menstrual cup, punch down or 7-fold fold are frequently used by the users, and when folded using these methods, it is preferable that the flexible portion 110 be formed to include the boundary point between the first receiving portion 110b and the second receiving portion 110c, and when folded using the diamond method, etc., to include a portion of the first receiving portion 100b, which is a location of the outermost side among the edges folded, and the flexible portion 110 may be formed in a curved plate shape across the first receiving portion 100b and the second receiving portion 100c, as shown in FIGS. 3 to 12.

A difference in the restoring force of the cup body 100 occurs according to an area of the flexible portion 110, and it is preferable that the flexible portion 110 be formed to have a certain level of restoring force after being folded. As an example, the flexible portion 110 may preferably be formed to have a length that is less than 2/3 of a total length of the cup body 100 and a perimeter or circumference that is less than 1/2 of a total circumference of the cup body 100 in a plan view. As a specific example, when the total length of the cup body 100 from the bottom of the cup body 100 to the rim portion 100a is 5 cm, the flexible portion 110 may be formed to have a length of 2 to 3 cm, and in a plan view, a circumference thereof may be formed at an angle of about 90 to 150° formed by the edges on both sides with a center of the cup body 100.

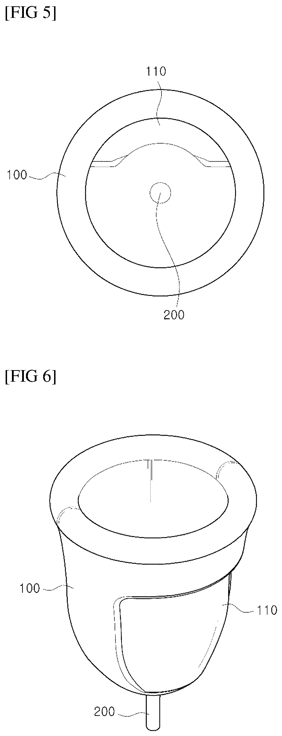

As described above, the flexible portion 110 may be manufactured separately from other parts or portions of the cup body 100 and then combined to form one body, it is preferable that they are formed integrally with the same material as other parts of the cup body 100 during production in terms of ease of manufacturing and safety in use. Accordingly, the flexible portion 110 may be formed as the inner peripheral surface of the cup body 100 is recessed more than other parts, as shown in FIGS. 3 to 5. The flexible portion 110 may also be formed as an outer peripheral surface of the cup body 100 is recessed more than other parts, as shown in FIGS. 6 to 9.

The menstrual cup in which the flexible portion 110 is formed as the inner peripheral surface of the cup body 100 is recessed, may receive or collect there inside more menstrual blood than the menstrual cup in which the flexible portion 110 is formed as the outer peripheral surface of the cup body 100 is recessed. Further, the menstrual cup in which the flexible portion 110 is formed as the outer peripheral surface of the cup body 100 is recessed enables the user to intuitively know where the folding part is and may demonstrate higher productivity than the menstrual cup in which the flexible portion 110 is formed as the inner peripheral surface of the cup body 100 is recessed.

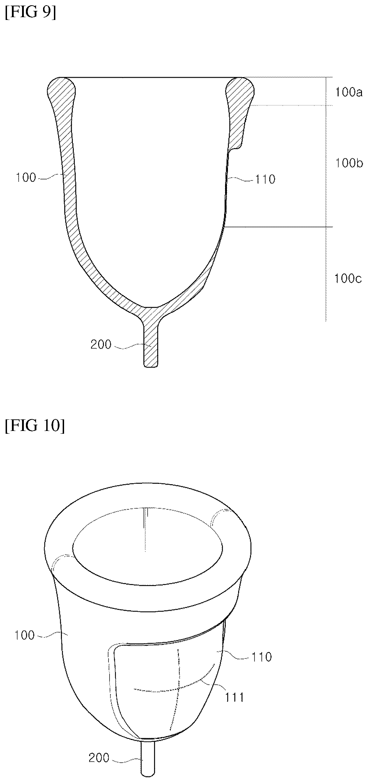

Meanwhile, when the flexible portion 110 is formed relatively thin compared to other portions and in a curved plate form, an elastic reinforced rim 111 may be additionally provided in order to improve elastic resilience or elastic restoring force with respect to corresponding parts or portions. The elastic reinforced rim 111, for example, may be of a material having a greater elasticity than a material constituting the flexible portion 110 or may be of the same material as the flexible portion 110 but of greater thickness than the flexible portion 110, and may be formed in a straight shape. This elastic reinforced rim 111 may be provided on the flexible portion 110 in the longitudinal or/and transverse direction as shown in FIG. 10.

As described above, in the menstrual cup of the present invention, when the cup body 100 is folded to be inserted into a woman's vagina, the flexible portion 110 that is relatively thin compared to other portions are crumpled, and the flexible portion 110, which is an outermost point of the edges formed as the cup body 100 is folded does not protrude sharply or pointedly, as shown in FIGS. 11 and 12, pain is not caused during the insertion into the vagina, and thus, discomfort and a sense of reluctance may be relieved.

## Claims

1. A menstrual cup comprising:
a cup body (100) formed in a shape of a bell with a hollow and an opening facing upward,
wherein the cup body (100) is formed with a flexible portion (110),
the flexible portion (110) comprising a point located at an outermost side among edges formed when the cup body (100) is folded and having a small thickness relative to other portions.

2. The menstrual cup of claim 1, wherein the cup body (100) comprises:
a rim portion (100a) at an upper end;
a first receiving portion (100b) extending downward from the rim portion (100a) in a shape of a cylinder which is open from top to bottom; and
a second receiving portion (100c) extending downward from the first receiving portion (100b) in a shape of a hemisphere which opens upward;
wherein the flexible portion (110) is formed to comprise a boundary point between the first receiving portion (100b) and the second receiving portion (100c).

3. The menstrual cup of claim 2, wherein the flexible portion(110) is formed:
in a shape of a curved plate across the first receiving portion (100b) and the second receiving portion (100c);
to have a length of less than 2/3 of a total length of the cup body (100); and
to have a circumference of less than 1/2 of a total circumference of the cup body (100), in a plan view.

4. The menstrual cup of claim 1, wherein the flexible portion (110) is formed with an inner peripheral surface of the cup body (100) recessed more than other portions.

5. The menstrual cup of claim 1, wherein the flexible portion (110) is formed with an outer peripheral surface of the cup body (100) recessed more than other portions.
